# EUROPÄISCHE PATENTANMELDUNG

(11) **EP 4 186 577 A1**
(43) Veröffentlichungstag der Anmeldung: **31.05.2023**
(21) Anmeldenummer: 21210430.1
(22) Anmeldetag: 25.11.2021
(51) Int. Cl.: B01D 19/00, A61M 5/36

(54) **VORRICHTUNG UND VERFAHREN ZUM ABTRENNEN VON GASBLASEN AUS EINEM FLÜSSIGKEITSSTROM**

(71) Anmelder: Medtron AG, 66128 Saarbrücken (DE)
(72) Erfinder: ALTMEYER, Hanno, 66538 Neunkirchen (DE); KREBER, Stefan, 66113 Saarbrüchen (DE); SCHWEIGMANN, Michael, 66482 Zweibrücken (DE)
(74) Vertreter: Wagner Albiger & Partner Patentanwälte mbB

(57) **Zusammenfassung**

Die Erfindung betrifft eine Vorrichtung zum Abtrennen von Gasblasen aus einem Flüssigkeitsstrom, umfassend eine Entgasungskammer (1) mit Eintrittskanal (10) und Austrittskanal (12) für die Durchleitung des Flüssigkeitsstromes und einer mit der Entgasungskammer (1) kommunizierenden Sammelkammer (112) mit Gasaustrittsöffnung (113) für die Gasblasen, wobei die Entgasungskammer (1) einen den Eintrittskanal (10) mit dem Austrittskanal (12) in einer Durchströmrichtung verbindenden und in einer vertikalen Ebene bogenförmig verlaufenden Ringkanal (11) umfasst und im vertikal oberen Bereich des Ringkanals (11) die Sammelkammer angeordnet ist. Es wird auch ein entsprechendes Verfahren zum Entfernen von Gasblasen aus einem Flüssigkeitsstrom angegeben.

## Beschreibung

Die Erfindung betrifft eine Vorrichtung zum Abtrennen von Gasblasen aus einem Flüssigkeitsstrom, umfassend eine Entgasungskammer mit Eintrittskanal und Austrittskanal für die Durchleitung des Flüssigkeitsstromes und einer mit der Entgasungskammer kommunizierenden Sammelkammer mit Gasaustrittsöffnung für die Gasblasen. Die Erfindung betrifft ferner auch ein Verfahren zum Abtrennen von Gasblasen aus einem Flüssigkeitsstrom.

Derartige Vorrichtungen und Verfahren sind bekannt und dienen dazu, unerwünschte Gasanteile in Form von in einem Flüssigkeitsstrom mitgeführten Gasblasen abzutrennen und gesondert vom Flüssigkeitsstrom abzuführen. Beispielsweise bei medizinischen Anwendungen, bei denen einem Patienten eine aus einem Injektor zugeführte Flüssigkeit in ein Blutgefäß injiziert wird, kommt einer solchen Abtrennung von Gasblasen besondere Bedeutung zu, da eine Injektion von Gasblasen in den Blutkreislauf unter Umständen lebensbedrohliche Komplikationen für den Patienten nach sich zieht. Beispiele derartiger mit Injektoren verabreichter Flüssigkeitsströme umfassen Kontrastmittel- und/oder Kochsalzlösungsinjektionen im Rahmen einer bildgebenden medizinischen Untersuchung, zum Beispiel Computertomografie (CT) oder Magnetresonanztomografie (MRT).

In derartigen Anwendungsfällen wird eine Vorrichtung und ein Verfahren gewünscht, welche zuverlässig die Abtrennung der Gasblasen aus dem Flüssigkeitsstrom sicherstellen, von medizinischem Personal leicht gehandhabt werden können und überdies insbesondere bei Verwendung als Einmalartikel zu niedrigen Herstellungskosten gefertigt werden können, wobei im Umfeld eines Magnetresonanztomografen überdies die Verwendung von metallischen Werkstoffen wegen der vorherrschenden starken Magnetfelder vermieden werden muss.

Aufgabe der Erfindung ist es, eine Vorrichtung und ein Verfahren der eingangs genannten Art vorzuschlagen, die insbesondere für die geschilderten Anwendungszwecke im medizinischen Umfeld geeignet sind.

Zur Lösung der gestellten Aufgabe wird erfindungsgemäß eine Vorrichtung gemäß den Merkmalen des Patentanspruchs 1 vorgeschlagen. Ein Verfahren zur Lösung der gestellten Aufgabe ist Gegenstand des Patentanspruchs 8.

Vorteilhafte Ausgestaltungen und Weiterbildungen der Erfindung sind Gegenstand der jeweils abhängigen Ansprüche.

Der erfindungsgemäße Vorschlag sieht eine Vorrichtung vor, bei der die Entgasungskammer einen den Eintrittskanal mit dem Austrittskanal in einer Durchströmrichtung verbindenden und in einer vertikalen Ebene bogenförmig, vorzugsweise kreisbogenförmig verlaufenden Ringkanal umfasst und im vertikal oberen Bereich des Ringkanals die Sammelkammer angeordnet ist.

Es hat sich im Rahmen der Erfindung gezeigt, dass mit einem solchen, in einer vertikalen Ebene angeordneten und den Eintrittskanal mit dem Austrittskanal bogenförmig verbindenden Ringkanal eine besonders effektive Abtrennung von mitgeführten Gasblasen aus dem Flüssigkeitsstrom bewirkt werden kann.

Dies ist darauf zurückzuführen, dass der über den Eintrittskanal zugeführte und Gasblasen enthaltende Flüssigkeitsstrom in dem in Durchströmrichtung anschließenden Ringkanal mit einer spezifischen Mindestfließgeschwindigkeit entsprechend des Verlaufs desselben bogenförmig in der vertikalen Ebene umgelenkt wird, wobei sich über die Breite des Strömungsquerschnittes durch den Ringkanal ein Geschwindigkeitsgradient des Flüssigkeitsstroms und der mitgeführten Gasblasen einstellt. In äußeren Bereichen des kreisbogenförmig verlaufenden Ringkanals, d. h. benachbart zum Außendurchmesser des Ringkanals, treten höhere Strömungsgeschwindigkeiten des Flüssigkeitsstroms einschließlich der mitgeführten Gasblasen auf als in den inneren Bereichen des bogenförmig verlaufenden Ringkanals, d. h. benachbart zum Innendurchmesser des Ringkanals. Dabei ist zu beobachten, dass mitgeführte großvolumige Gasblasen aufgrund des in einer vertikalen Ebene bogenförmig verlaufenden Ringkanals infolge der geringeren Dichte gegenüber dem Flüssigkeitsstrom rasch zum Außendurchmesser des Ringkanals vertikal nach oben aufsteigen und dort in die im vertikal oberen Bereich des Ringkanals angeordnete Sammelkammer übertreten. Von dort können die aus dem Flüssigkeitsstrom austretenden Gasblasen über eine entsprechende Gasaustrittsöffnung getrennt vom Flüssigkeitsstrom abgeführt werden.

Der solchermaßen von den großvolumigen Gasblasen befreite Flüssigkeitsstrom wird im Ringkanal in Richtung Austrittskanal geführt.

Zugleich werden aber durch den vorherrschenden Geschwindigkeitsgradienten des Flüssigkeitsstromes die noch enthaltenen kleinvolumigen Gasblasen, die aufgrund ihres geringen Volumens nicht mit großer Geschwindigkeit im Flüssigkeitsstrom aufsteigen und der Sammelkammer unmittelbar zugeführt werden, innerhalb des bogenförmig verlaufenden Ringkanals durch die vorherrschende Kombination aus Zentrifugalkraft und Dichteunterschied radial nach innen abgelenkt, wodurch ihre Strömungsgeschwindigkeit in Durchströmrichtung weiter abnimmt. Sobald die kleinvolumigen Gasblasen in Kontakt mit der den Innendurchmesser des bogenförmig verlaufenden Ringkanals bildenden Oberfläche desselben kommen, sammeln sie sich an dieser Oberfläche und agglomerieren allmählich zu großvolumigen, aus einer Vielzahl kleinvolumiger Gasblasen zusammengesetzten Gasblasen, die letztlich ein so großes Volumen aufweisen, dass sie in der Lage sind, selbsttätig vertikal nach oben aufzusteigen und in die Sammelkammer überzutreten.

Es konnte anhand von Versuchen nachgewiesen werden, dass auf diese Weise unterschiedlichste Volumengrößen von Gasblasen in einem Flüssigkeitsstrom zuverlässig entfernt werden können, wobei die Effektivität umso größer ist, je höher die Strömungsgeschwindigkeit des Flüssigkeitsstromes durch den Ringkanal ist. Dies kommt medizinischen Anwendungen beispielsweise mittels Injektion sehr entgegen, da zum Beispiel bei angiographischen Untersuchungen mit Injektionsdrücken von bis zu 83 bar und entsprechend hohen Strömungsgeschwindigkeiten des Flüssigkeitsstroms, etwa mit bis zu 40 ml/s gearbeitet wird.

Die Unterscheidung in großvolumige und kleinvolumige Gasblasen ist abhängig von den jeweiligen Dichteverhältnissen. Unter großvolumigen Gasblasen versteht die Erfindung solche Gasblasen, die selbsttätig im Flüssigkeitsstrom aufsteigen und in die Sammelkammer übertreten. Reicht die Auftriebskraft kleinerer Gasblasen für ein solches selbsttätiges Aufsteigen in die Sammelkammer nicht aus, handelt es sich im Sinne dieser Erfindung um kleinvolumige Gasblasen. Die Untergrenze der kleinvolumigen Gasblasen liegt in einer Größenordnung, die ohne Gefahr in den Blutkreislauf eines Patienten injiziert werden können.

Zur weiteren Steigerung der Strömungsgeschwindigkeit ist nach einem Vorschlag der Erfindung vorgesehen, dass sich der Ringkanal in Durchströmrichtung in seinem Querschnitt verjüngt, sodass ausgehend vom Eintrittskanal bis zum Austrittskanal die Strömungsgeschwindigkeit erhöht wird.

Nach einem weiteren Vorschlag der Erfindung verläuft der Ringkanal durchgängig über 360° in der vertikalen Ebene, wobei der Eintrittskanal tangential und in Durchströmrichtung vor der Sammelkammer in den Ringkanal einmündet und der Austrittskanal in Durchströmrichtung betrachtet nach der Sammelkammer und vor der Einmündung des Eintrittskanals radial nach außen aus dem Ringkanal herausgeführt ist. Eine solche Anordnung ermöglicht es, lediglich eine Teilmenge des im Ringkanal geführten Flüssigkeitsstromes nach Zuführung über den Eintrittskanal sogleich aus dem Austrittskanal wieder abzuführen. Der verbleibende Rest der im Ringkanal geführten Flüssigkeit wird hingegen über einen vollständigen 360°-Umlauf im Ringkanal geführt und durchläuft diesen mindestens zweifach, bis er letztlich über den Austrittskanal abgeführt wird. Durch eine solche Anordnung kann sich erfindungsgemäß die Effizienz der Abtrennung von mitgeführten Gasblasen nochmals deutlich steigern.

Um eine homogene Durchmischung der über einen vollständigen Umlauf im Ringkanal geführten Teilmenge des Flüssigkeitsstroms mit neu über den Eintrittskanal in den Ringkanal zugeführten Flüssigkeitsstrom zu gewährleisten, kann überdies in Durchströmrichtung betrachtet vorzugsweise unmittelbar vor der Einmündung des Eintrittskanals eine Überströmstufe ausgebildet sein, die an die Einmündung des Eintrittskanals angrenzt und den im vollständigen Umlauf geführten Flüssigkeitsstrom geschichtet ober- oder unterhalb des über den Eintrittskanal zugeführten Flüssigkeitsstroms mit diesem zusammenführt.

Nach einem weiteren Vorschlag der Erfindung wird der Ringkanal von einer im Wesentlichen bogenförmigen Innenwand und einer konzentrisch zu dieser angeordneten bogenförmigen Außenwand begrenzt, wobei Letztere im Bereich der Einmündungen von Eintritts- und Austrittskanal sowie der Sammelkammer unterbrochen ist. Die bogenförmige Innenwand hingegen ist bevorzugt kontinuierlich ausgeführt.

Nach einem weiteren Vorschlag der Erfindung ist die vorangehend erläuterte Entgasungskammer in einem Einsatzgehäuse angeordnet, welches beispielsweise als auswechselbares Einwegteil in einem entsprechenden Aufnahmegehäuse eines medizinischen Injektors einsetzbar und im Bereich des Eintrittskanals, des Austrittskanals sowie der Gasaustrittsöffnung mit entsprechenden Anschlüssen des medizinischen Injektors bei Einsetzen in das Aufnahmegehäuse verbunden wird.

Das im Rahmen der Erfindung vorgeschlagene Verfahren zum Abtrennen von Gasblasen aus einem Flüssigkeitsstrom beruht darauf, dass der Flüssigkeitsstrom in einem Ringkanal in einer Durchströmrichtung bogenförmig, vorzugsweise im wesentlichen kreisbogenförmig in einer vertikalen Ebene ausgehend von einem Eintrittskanal zu einem Austrittskanal geführt wird. Erfindungsgemäß ist dabei vorgesehen, dass infolge der in vertikaler Ebene erfolgenden bogenförmigen Führung des Flüssigkeitsstromes mitsamt der mitgeführten Gasblasen im Ringkanal großvolumige Gasblasen in vertikaler Richtung zu einer Entgasungskammer aufsteigen und von dort abgeführt werden, während die verbleibenden kleinvolumigen Gasblasen radial nach innen abgelenkt werden und sich im Bereich einer Innenwandlung des Ringkanals anlagern und zu großvolumigen Gasblasen agglomerieren und dann zu der Entgasungskammer aufsteigen und abgeführt werden.

Erfindungsgemäß kann dabei vorgesehen sein, dass der Flüssigkeitsstrom in Durchströmrichtung beschleunigt wird, beispielsweise durch eine allmähliche Veränderung des zur Verfügung stehenden Strömungsquerschnittes im Ringkanal.

Schließlich kann vorgesehen sein, dass nur eine Teilmenge des im Ringkanal geführten Flüssigkeitsstromes unmittelbar über den Austrittskanal aus dem Ringkanal abgeführt wird, wobei die verbleibende Teilmenge für einen vollständigen Umlauf im Ringkanal verbleibt und diesen erneut durchströmt.

Weitere Ausgestaltungen und Einzelheiten der erfindungsgemäßen Vorrichtung und des Verfahrens werden nachfolgend anhand eines Ausführungsbeispiels in der Zeichnung erläutert. Es zeigen:

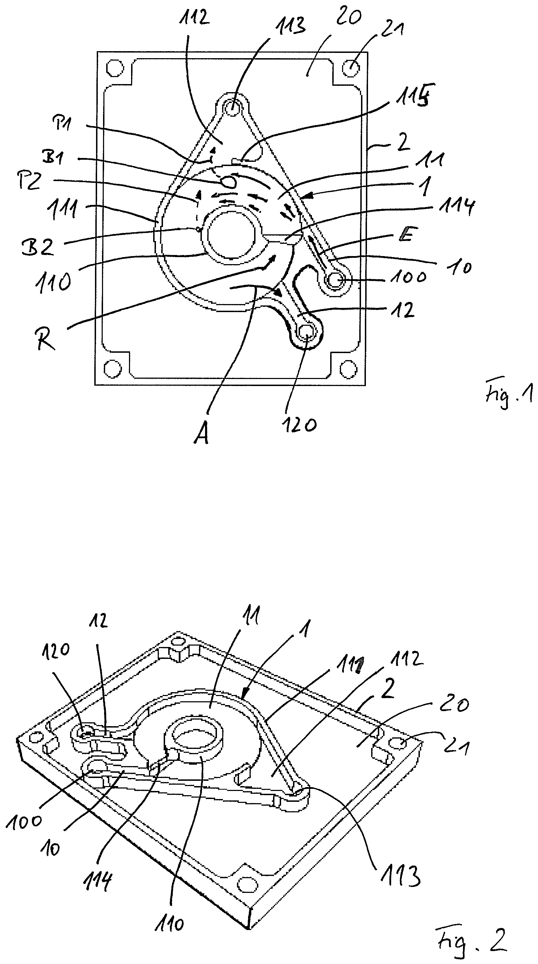

- Figur 1: eine Aufsicht auf eine erfindungsgemäße Vorrichtung;
- Figur 2: die Vorrichtung gemäß Figur 1 in einer perspektivischen Darstellung.

Aus den Figuren ist eine Vorrichtung zum Abtrennen von Gasblasen aus einem Flüssigkeitsstrom ersichtlich, die beispielsweise einstückig aus einem spritzgegossenen, gedruckten oder gefrästen Kunststoff als Einwegteil hergestellt werden kann und sich insbesondere für medizinische Anwendungen unter entsprechenden Hygienebedingungen eignet.

Wesentlicher Bestandteil der aus den Figuren ersichtlichen Vorrichtung ist eine nachfolgend in weiteren Einzelheiten erläuterte Entgasungskammer 1, die von einem hier quaderförmig ausgebildeten Einsatzgehäuse 2 umgeben ist, welches eine sich in der dargestellten Einbauposition in einer vertikalen Ebene erstreckende Rückwand 20 sowie eine hier abgenommene und in Bohrungen 21 fixierte vorderseitige Wand umfasst, zwischen denen die Entgasungskammer 1 angeordnet ist. In dieser gezeigten vertikalen Orientierung wird die Vorrichtung genutzt.

Diese Entgasungskammer 1 umfasst eine die Rückwand 20 durchsetzende Eintrittsöffnung 100 für einen Flüssigkeitsstrom, beispielsweise im Falle eines medizinischen Injektors eine von diesem in Richtung auf einen Patienten injizierte Kontrastmittel- und/oder Kochsalzlösung, die gemäß Pfeil E über einen Eintrittskanal 10 in die Entgasungskammer 1 eintritt. Anschließend an den Eintrittskanal 10 umfasst die Entgasungskammer 1 einen mit dem Eintrittskanal 10 kommunizierenden Ringkanal 11, der im dargestellten Ausführungsbeispiel kreisbogenförmig in der vertikalen Ebene zunächst aufwärts und dann abwärts verläuft. Die Einmündung des Eintrittskanals 10 in den demgegenüber mit deutlich vergrößertem Strömungsquerschnitt ausgebildeten Ringkanal 11 erfolgt tangential in einer etwa 3:00 Uhr Position und ist vertikal aufwärts gerichtet.

Der Ringkanal 11 wird einerseits von einer ununterbrochenen zylindrischen Innenwand 110 sowie einer konzentrisch hierzu angeordneten Außenwand 111 begrenzt, wobei letztere einerseits von der Einmündung des Eintrittskanals 10, andererseits auch von nachfolgend noch näher erläuterten Einmündungen eines Austrittskanals 12 sowie im vertikal oberen Bereich von einer Sammelkammer 112 unterbrochen ist, die insoweit zwischen den Einmündungen von Eintrittskanal 10 und Austrittskanal 12 positioniert ist.

Die Sammelkammer 112 ist etwa kuppelförmig im vertikal oberen Bereich des Ringkanals 11 angrenzend an dessen Außenumfang angeordnet und wird über einen Steg 115 vom Ringkanal 11 unter Belastung einer Öffnung abgeteilt. Im vertikal unteren Bereich etwa in einer 5:00 Uhr Stellung führt der Austrittskanal 12 radial nach außen aus dem Ringkanal 11 heraus. Der Austrittskanal 12 kommuniziert über eine aus dem Einsatzteil 2 und dessen Rückwand 20 herausführende Austrittsbohrung 120 mit einer hier nicht dargestellten Anschlussleitung zum Patienten.

Gleichermaßen ist auch die vertikal oberhalb angeordnete Sammelkammer 112 mit einer die Rückwand 20 durchsetzenden Gasaustrittsöffnung 113 versehen.

Bei Beauftragung der Entgasungskammer 1 mit dem über den Eintrittskanal 10 zugeführten Flüssigkeitsstrom tritt dieser in den Ringkanal 11 ein und wird infolgedessen in vertikaler Ebene kreisbogenförmig im Verlauf des Ringkanals 11 auf eine sich in einer vertikalen Ebene erstreckende Kreisbahn gezwungen. Mitgeführte großvolumige Gasblasen B1, die ein solches Volumen aufweisen, dass sie selbsttätig infolge des Dichteunterschiedes im Flüssigkeitsstrom aufsteigen können, gelangen gemäß Pfeil P1 unmittelbar in die vertikal oberhalb des Ringkanals 11 angeordnete Sammelkammer 112 und werden über die Gasaustrittsöffnung 113 abgeführt. Der Flüssigkeitsstrom hingegen wird entlang des Steges 115 weiter auf der Kreisbahn und in einer Durchströmrichtung entlang des Ringkanals 11 in Richtung des Austrittskanals 12 geführt.

Mitgeführte kleinvolumigen Gasblasen B2, deren Auftriebsvermögen nicht ausreicht, um unmittelbar in die Sammelkammer 112 aufzusteigen, werden durch einen sich im Ringkanal 11 einstellenden Geschwindigkeitsgradienten der Fließgeschwindigkeit des Flüssigkeitsstromes an die Innenwand 110 des Ringkanals 11 gedrängt, an der sie letztlich anhaften. Der Geschwindigkeitsgradient ist durch unterschiedliche Pfeillängen im Ringkanal 11 dargestellt, wobei im radial äußeren Bereich des Ringkanals 11 größere Strömungsgeschwindigkeiten als im radial inneren Bereich des Ringkanals vorherrschen und im Bereich der Innenwand 110 fast zum Erliegen kommen. Demzufolge sammeln sich die kleinvolumigen Gasblasen B2 allmählich auf der Innenwand 110 an und fließen zusammen, d. h. sie agglomerieren, wobei sie an Volumen zunehmen, bis sie letztlich ein solches Gesamtvolumen annehmen, dass sie selbsttätig gemäß Pfeil P1 in die Sammelkammer 112 aufsteigen können.

Der beim Umlauf durch die Ringkammer 11 von Gasblasen befreite Flüssigkeitsstrom wird sodann nach einem etwa ¾-Umlauf zumindest zu einem Teil gemäß Pfeil A über den radial nach außen führenden Austrittskanal 12 und die sich anschließende Austrittsbohrung 120 abgeführt, wohingegen der verbleibende Teil des Flüssigkeitsstromes gemäß Pfeil R einen vollständigen Umlauf im Ringkanal 11 durchläuft und erneut mit über den Eintrittskanal 10 zugeführtem Flüssigkeitsstrom zusammentrifft, um sodann mit diesem gemeinsam einen erneuten Umlauf im Ringkanal 11 unter Abtrennung weiterer Gasblasen zu absolvieren.

Um das Auftreten von Turbulenzen im Bereich des Zusammenfließens des einen vollständigen Umlauf gemäß Pfeil R absolvierenden Teilstroms sowie gemäß Pfeil E über den Eintrittskanal 10 zugeführten weiteren Flüssigkeitsstromes zu verhindern, ist in Durchströmrichtung betrachtet unmittelbar vor der Einmündung des Eintrittskanals 10 in den Ringkanal 11 eine Überströmstufe 114 im Ringkanal 11 ausgebildet, die dafür sorgt, dass sich zwei Schichten im Ringkanal 11 bilden, nämlich einerseits eine Schicht aus dem über den Eintrittskanal 10 gemäß Pfeil E zuströmenden Flüssigkeitsstrom und darauf eine weitere Schicht aus dem Teilstrom gemäß Pfeil R, der im Ringkanal 11 einen vollständigen Umlauf absolviert.

Es hat sich gezeigt, dass mit einer solchen einfachen und ohne bewegliche Teile auskommenden Vorrichtung ein Flüssigkeitsstrom insbesondere bei hohen Strömungsgeschwindigkeiten zuverlässig von mitgeführten Gasblasen getrennt werden kann, wobei die dafür erforderliche Energie allein aus der Strömungsgeschwindigkeit des Flüssigkeitsstroms gewonnen werden kann.

## Patentansprüche

1. Vorrichtung zum Abtrennen von Gasblasen aus einem Flüssigkeitsstrom, umfassend eine Entgasungskammer (1) mit Eintrittskanal (10) und Austrittskanal (12) für die Durchleitung des Flüssigkeitsstromes und einer mit der Entgasungskammer (1) kommunizierenden Sammelkammer (112) mit Gasaustrittsöffnung (113) für die Gasblasen, wobei die Entgasungskammer (1) einen den Eintrittskanal (10) mit dem Austrittskanal (12) in einer Durchströmrichtung verbindenden und in einer vertikalen Ebene bogenförmig verlaufenden Ringkanal (11) umfasst und im vertikal oberen Bereich des Ringkanals (11) die Sammelkammer angeordnet ist.

2. Vorrichtung nach Anspruch 1, **dadurch gekennzeichnet, dass** der Ringkanal (11) in der vertikalen Ebene kreisbogenförmig verläuft.

3. Vorrichtung nach Anspruch 1 oder 2, **dadurch gekennzeichnet, dass** der Ringkanal (11) sich in Durchströmrichtung in seinem Querschnitt verjüngt.

4. Vorrichtung nach einem der Ansprüche 1 bis 3, **dadurch gekennzeichnet, dass** der Ringkanal (11) durchgängig über 360° verläuft und der Eintrittskanal (10) tangential in den Ringkanal (11) einmündet und der Austrittskanal (12) in Durchströmrichtung betrachtet vor der Einmündung des Eintrittskanals (10) radial nach außen aus dem Ringkanal (11) herausgeführt ist.

5. Vorrichtung nach Anspruch 4, **dadurch gekennzeichnet, dass** der Ringkanal (11) in Durchströmrichtung betrachtet vor der Einmündung des Eintrittskanals (10) mit einer Überströmstufe (114) ausgebildet ist, die an die Einmündung des Eintrittskanals (10) angrenzt.

6. Vorrichtung nach einem der Ansprüche 1 bis 5, **dadurch gekennzeichnet, dass** der Ringkanal (11) von einer im wesentlichen bogenförmigen Innenwand (110) und einer konzentrisch zu dieser angeordneten Außenwand (111) begrenzt ist, die im Bereich der Einmündungen von Eintritts- und Austrittskanal (10, 12) sowie der Sammelkammer (112) unterbrochen ist.

7. Vorrichtung nach einem der Ansprüche 1 bis 6, **dadurch gekennzeichnet, dass** die Entlüftungskammer (1) in einem Einsatzgehäuse (2) angeordnet ist.

8. Medizinischer Injektor zum Injizieren eines Flüssigkeitsstromes in einen Patienten, umfassend eine Vorrichtung gemäß einem der vorangehenden Ansprüche.

9. Verfahren zum Abtrennen von Gasblasen aus einem Flüssigkeitsstrom, bei welchem der die Gasblasen enthaltende Flüssigkeitsstrom in einem Ringkanal (11) in einer Durchströmrichtung bogenförmig in einer vertikalen Ebene und ausgehend von einem Eintrittskanal (10) zu einem Austrittskanal (12) geführt wird, wobei großvolumige Gasblasen in vertikaler Richtung zu einer Entgasungskammer (112) aufsteigen und abgeführt werden und die verbleibenden kleinvolumigen Gasblasen radial nach innen abgelenkt werden und sich im Bereich einer Innenwandung des Ringkanals (11) ansammeln und zu großvolumigen Gasblasen agglomerieren und zu der Entgasungskammer (112) aufsteigen und abgeführt werden.

10. Verfahren nach Anspruch 9, **dadurch gekennzeichnet, dass** der die Gasblasen enthaltende Flüssigkeitsstrom im Ringkanal (11) im wesentlichen kreisbogenförmig in der vertikalen Ebene geführt wird.

11. Verfahren nach Anspruch 9 oder 10, **dadurch gekennzeichnet, dass** der Flüssigkeitsstrom in Durchströmrichtung beschleunigt wird.

12. Verfahren nach einem der Ansprüche 9 bis 11, **dadurch gekennzeichnet, dass** nur eine Teilmenge des im Ringkanal (11) geführten Flüssigkeitsstromes über den Austrittskanal (12) aus dem Ringkanal (11) abgeführt wird und die verbleibende Teilmenge für einen vollständigen Umlauf im Ringkanal (11) verbleibt und diesen erneut durchströmt.
